(19) Europäisches Patentamt
European Patent Office
Office européen des brevets

(11) EP 4 254 582 A1

(12) **EUROPEAN PATENT APPLICATION**
published in accordance with Art. 153(4) EPC

(43) Date of publication:
**04.10.2023 Bulletin 2023/40**

(21) Application number: **21896847.7**

(22) Date of filing: **17.11.2021**

(51) International Patent Classification (IPC):
***H01M 10/0567*** *(2010.01)* ***H01M 4/58*** *(2010.01)*
***H01M 10/0525*** *(2010.01)*

(52) Cooperative Patent Classification (CPC):
**H01M 4/485; H01M 4/525; H01M 4/58; H01M 10/0525; H01M 10/0566; H01M 10/0567; H01M 10/058; H01M 10/42**

(86) International application number:
**PCT/CN2021/131060**

(87) International publication number:
**WO 2022/111346 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.11.2020 CN 202011375677**

(71) Applicant: **Shenzhen Capchem Technology Co., Ltd**
**Shenzhen, Guangdong 518118 (CN)**

(72) Inventors:
- **QIAN, Yunxian**
**Shenzhen, Guangdong 518118 (CN)**
- **HU, Shiguang**
**Shenzhen, Guangdong 518118 (CN)**
- **LI, Hongmei**
**Shenzhen, Guangdong 518118 (CN)**
- **YUAN, Xiaogang**
**Shenzhen, Guangdong 518118 (CN)**
- **DENG, Yonghong**
**Shenzhen, Guangdong 518118 (CN)**

(74) Representative: **De Arpe Tejero, Manuel**
**Arpe Patentes y Marcas**
**Alcalá, 26, 5ª Planta**
**28014 Madrid (ES)**

(54) **LITHIUM-ION BATTERY**

(57) The application relates to the technical field of lithium ion batteries and discloses a lithium ion battery. The lithium ion battery comprises a positive electrode, a negative electrode, a separator arranged between the positive electrode and negative electrode, and a non-aqueous electrolyte; an active material of the positive electrode comprises $LiFePO_4$, the non-aqueous electrolyte comprises an organic solvent, a lithium salt, vinylene carbonate and a compound represented by Formula (1), wherein $R_1$ is one or more of chain, cyclic and aromatic group with 2-20 carbon atoms, and a compacted density of the positive electrode material is more than 2g/cm$^3$. The lithium ion battery provided by the application may obviously improve the cycle and storage performances at high temperature, greatly improve the capacity retention rate and capacity recovery rate of battery, obviously reduce the thickness expansion rate after high-temperature storage, and meanwhile, greatly reduce the precipitation of iron ions.

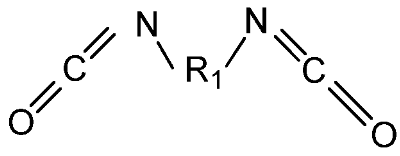

Formula (1)

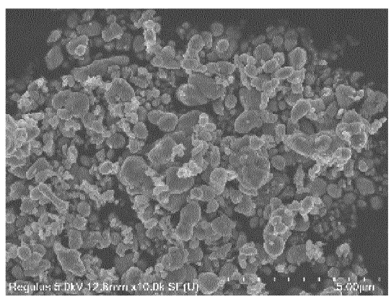

Fig. 1

EP 4 254 582 A1

## Description

### Technical field

The present application relates to the technical field of lithium ion batteries, in particular to a lithium iron phosphate battery.

### Background

lithium ion batteries are widely used in production and life because of their excellent performances. In recent years, with the continuous improvement of the driving range requirements of new energy vehicles, people have put forward higher requirements for the cycle life and safety of lithium ion batteries.
Compared with ternary battery, lithium iron phosphate battery has advantages in cycle life, safety and cost, but there is still a big gap between the energy density of lithium iron phosphate and that of ternary materials. Improving the compacted density and area density of positive and negative plates is one of the important means to improve the energy density of lithium iron phosphate battery. However, the lower the porosity of the electrode plate, the higher the requirement for electrolyte. Firstly, when the porosity is low, the electrolyte is difficult to penetrate into the electrode plate, resulting in insufficient liquid retention for battery manufacture, which further leads to obvious attenuation of the cycle performance of battery, and at the same time, there is also the problem of lithium precipitation. Secondly, the lack of liquid retention would also lead to the increase of contact internal resistance between electrolyte and electrode plate, which would affect the battery capacity and rate discharge performance.
Generally, there are two main solutions for developing electrolyte suitable for high-voltage solid electrode plate: one is to adopt a solvent with low viscosity to promote electrolyte infiltration and improve the cycle and rate performance of batteries; the other is to add additives to reduce impedance and promote cycle, thus improving the service life of battery. However, these two methods would lead to the deterioration of the high-temperature performance of electrolyte to a certain extent, and the phenomenon of gas expansion is also serious. Therefore, how to improve the high-temperature cycle and high-temperature storage performances of battery while ensuring that the battery does not precipitate lithium is a difficult problem faced with the electrolyte of high-voltage lithium iron phosphate batteries.

### Summary

The purpose of the present application is to overcome the problems of poor high-temperature performances and serious gas expansion of high-voltage lithium iron phosphate batteries in the prior art, and provide a lithium ion battery, which has the advantages of good cycle and storage performances at high temperature, and the like.
In order to achieve the above object, the present application provides a lithium ion battery, which includes a positive electrode, a negative electrode, a separator arranged between the positive electrode and the negative electrode, and a non-aqueous electrolyte; an active material of the positive electrode includes $LiFePO_4$, the non-aqueous electrolyte includes an organic solvent, a lithium salt, vinylene carbonate and a compound represented by Formula (1):

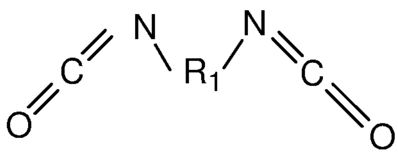

Formula (1)

in Formula (1), $R_1$ is one or more of chain, cyclic and aromatic group with 2-20 carbon atoms, and a compacted density of the positive electrode material is 2g/cm$^3$ or more.
Preferably, in Formula (1), $R_1$ is one or more of chain, cyclic and aromatic group with 3-18 carbon atoms.
More preferably, $R_1$ is selected from one or more of following structures, * represents binding position:

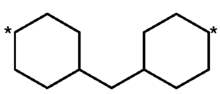,
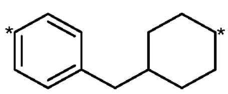,
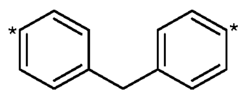,

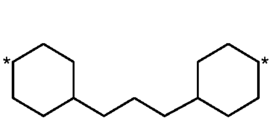, 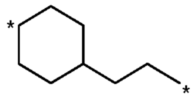, 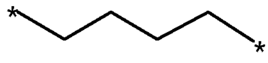,

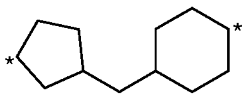, 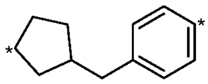, 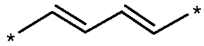,

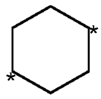, 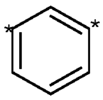 and 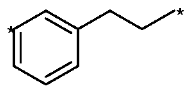.

Preferably, the compound represented by Formula (1) is selected from one or more of the following compounds:

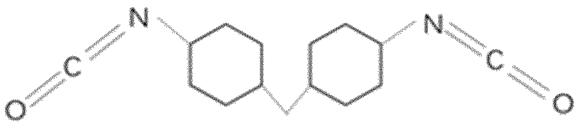

Compound 1,

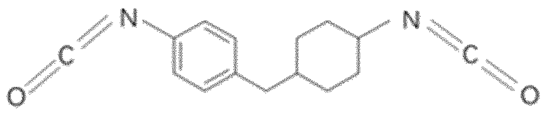

Compound 2,

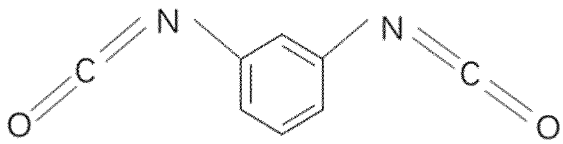

Compound 3,

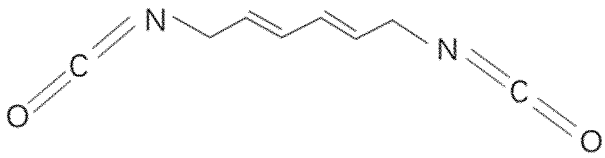

Compound 4,

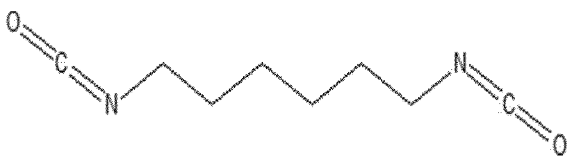

Compound 5,

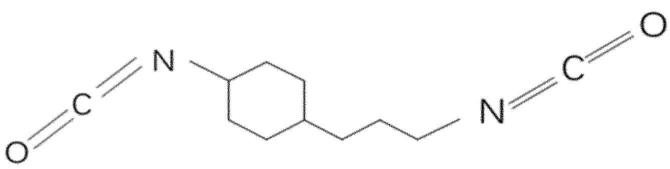

Compound 6,

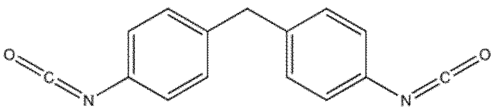

Compound 7

and

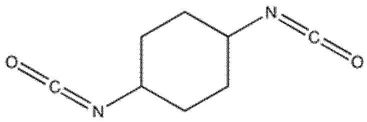

Compound 8.

Preferably, a content of the compound represented by Formula (1) in the non-aqueous electrolyte is 0.001-5 wt% based on a total weight of the non-aqueous electrolyte; more preferably, the content of the compound represented by Formula (1) in the non-aqueous electrolyte is 0.001-3 wt% based on the total weight of the non-aqueous electrolyte.

Preferably, the content of vinylene carbonate in the non-aqueous electrolyte is 0.1-5 wt% based on the total weight of the non-aqueous electrolyte; more preferably, the content of the vinylene carbonate in the non-aqueous electrolyte is 0.5-3 wt% based on the total weight of the non-aqueous electrolyte.

Preferably, the organic solvent is one or more of cyclic carbonate, linear carbonate, carboxylic ester and ether.

Preferably, the cyclic carbonate includes one or more of vinylene carbonate, propylene carbonate and ethylene carbonate.

Preferably, the linear carbonate includes one or more of dimethyl carbonate, diethyl carbonate and ethyl methyl carbonate.

Preferably, the carboxylic ester includes one or more of methyl acetate, ethyl acetate, methyl propionate, ethyl propionate, methyl butyrate, methyl isobutyrate, methyl trimethylacetate and ethyl trimethylacetate.

Preferably, the ether includes one or more of ethylene glycol dimethyl ether, 1,3-dioxolane and 1,1,2,2-tetrafluoroethyl-2,2,3,3-tetrafluoropropyl ether.

More preferably, the organic solvent is a mixture of ethylene carbonate and ethyl methyl carbonate.

Preferably, the lithium salt is selected from one or more of $LiPF_6$, $LiBF_4$, LiBOB, LiDFOB, $LiPO_2F_2$, $LiSbF_6$, $LiAsF_6$, $LiN(SO_2CF_3)_2$, $LiN(SO_2C_2F_5)_2$, $LiC(SO_2CF_3)_3$ and LiFSI; more preferably, the lithium salt is selected from one or more of $LiPF_6$, $LiPO_2F_2$ and LiFSI.

Preferably, the content of the lithium salt in the non-aqueous electrolyte is 0.1-2 mol/L; and more preferably, the content of the lithium salt in the non-aqueous electrolyte is 0.8-1.5 mol/L.

Preferably, the non-aqueous electrolyte further includes other additives, and the other additives are 1,3-propane sultone, 1,4-butane sultone, methylene methane disulfonate, fluoroethylene carbonate, ethylene sulfate, methylene methyl disulfonate, lithium difluorophosphate, lithium difluoro(oxalato)borate, tris (trimethylsilane) phosphate, propene sultone, fluorobenzene and vinyl ethylenecarbonate; preferably, the other additives are one or more of lithium difluorophosphate, ethylene sulfate and methylene methanedisulfonate.

Preferably, the content of vinylene carbonate in the non-aqueous electrolyte is 0.1-5 wt% based on the total weight of the non-aqueous electrolyte; more preferably, the content of the vinylene carbonate in the non-aqueous electrolyte is 0.1-3 wt% based on the total weight of the non-aqueous electrolyte.

More preferably, the compacted density of the material of the positive electrode is 2-3g/$cm^3$.

Preferably,a compacted density of a material of the negative electrode is above 1.3g/$cm^3$; and more preferably, the compacted density of the material of the negative electrode is 1.3-1.8g/$cm^3$.

Preferably, a voltage range of the lithium ion battery is 2-4V; more preferably, the voltage range of the lithium ion battery is 2-3.65 V.

Preferably, an liquid injection coefficient of the lithium ion battery is 2-7g/Ah; more preferably, the liquid injection coefficient of the lithium ion battery is 3-5g/Ah.

According to the above technical solutions, the cycle and storage performances of lithium iron phosphate battery at high temperature may be significantly improved, the capacity retention rate and capacity recovery rate of battery may be greatly improved, the thickness expansion rate of lithium iron phosphate battery after high temperature storage may be significantly reduced, and at the same time, the precipitation of iron ions may be greatly reduced.

Brief description of drawings

Fig. 1 is a SEM image of negative electrode of lithium ion battery after 2500 cycles at 45°C, according to Embodiment 7 of the present application.

Fig. 2 is a SEM image of negative electrode of lithium ion battery after 2500 cycles at 45°C, according to Comparative example 1 of the present application.

Detailed description of preferred embodiments

The range values including endpoints disclosed herein are not limited to the precise range or value, these ranges or values should be understood to include values close to these ranges or values. For numerical ranges, one or more new numerical ranges may be obtained by combining the endpoint values of various ranges and individual point values. Individual point values and these numerical ranges should be regarded as specifically disclosed in the present application.

The present application provides a lithium ion battery, which includes a positive electrode, a negative electrode, a separator arranged between the positive electrode and the negative electrode, and a non-aqueous electrolyte; an active material of the positive electrode includes $LiFePO_4$, the non-aqueous electrolyte includes an organic solvent, a lithium salt, vinylene carbonate and a compound represented by Formula (1):

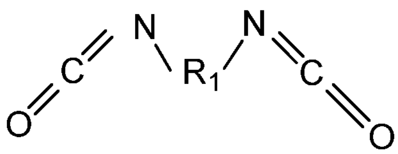

Formula (1)

in Formula (1), $R_1$ is one or more of chain, cyclic and aromatic group with 2-20 carbon atoms, and a compacted density of the positive electrode material is 2g/cm$^3$ or more.

Through a lot of research and experiments, the inventors of the present application found that for a lithium ion battery with $LiFePO_4$ as positive active material, when the non-aqueous electrolyte of the lithium ion battery contains both vinylene carbonate (VC) and the compound represented by Formula (1), and the compacted density of the positive material is in the range defined by the present application, the cycle and storage performances of the lithium iron phosphate battery at high temperature may be significantly improved, the size expansion rate of the lithium iron phosphate battery after high-temperature storage may be greatly reduced, and the precipitation of iron ions may also be reduced.

The reason may be: due to the precipitation of iron ions in positive electrode material of lithium iron phosphate battery system, the precipitated iron ions would be deposited on the surface of negative electrode, blocking the channel of lithium ion intercalation or de-intercalation, resulting in a significant decrease in the rate of lithium ion intercalation or de-intercalation on the surface of the negative electrode, thus forming lithium dendrites on the surface of negative electrode. The reaction of lithium dendrites with electrolyte would also generate a large amount of gas, which would expand the battery size and even cause the battery to cycle diving in severe cases. The phenomenon of iron ion precipitation is more serious under high pressure, and the compound represented by Formula (1) can play a complexing role on iron ion, and through the synergistic effect with vinylene carbonate, iron ion precipitated from the positive electrode and deposited on the negative electrode may be inhibited within the range of compacted density of the positive electrode defined by the present application, so that the occurrence of side reactions and the loss of electrolyte are reduced, and thus the high-temperature cycle and storage performances of the battery are significantly improved.

According to the present application, preferably, in Formula (1), $R_1$ is one or more of chain, cyclic and aromatic groups with 3-18 carbon atoms; more preferably, $R_1$ is selected from one or more of the following structures, wherein * represents a binding position (i.e., an atom connected to the atom N in Formula (1)):

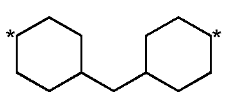, 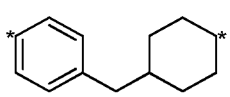, 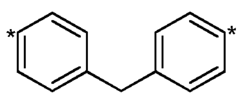,

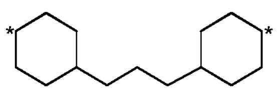, 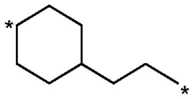, 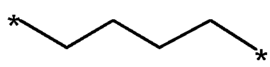,

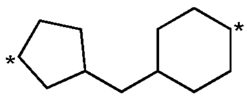, 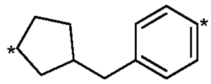, 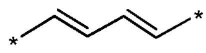,

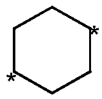, 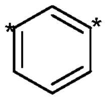 and 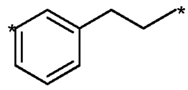.

In the present application, preferably, the compound represented by Formula (1) is selected from one or more of the following compounds:

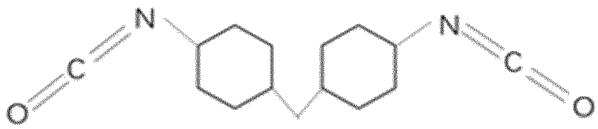 Compound 1,

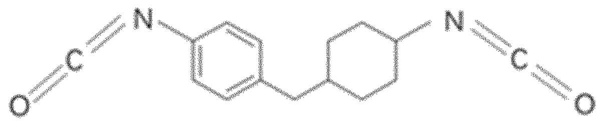 Compound 2,

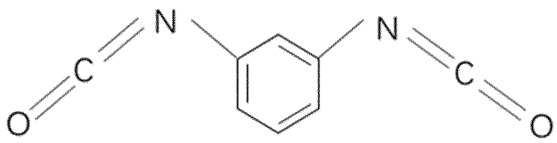 Compound 3,

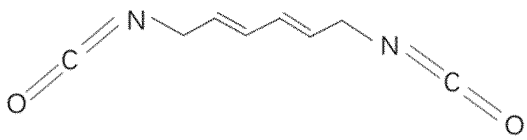 Compound 4,

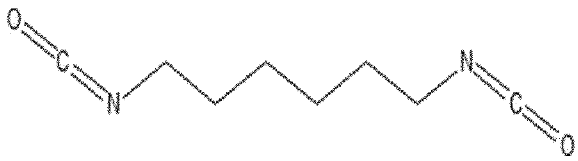 Compound 5,

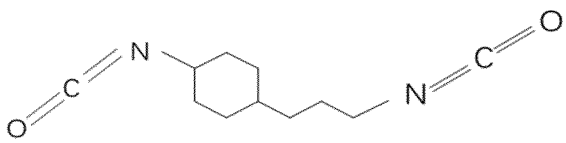 Compound 6,

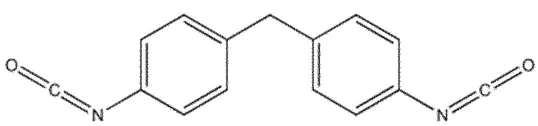 Compound 7

and

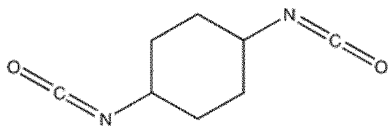

Compound 8.

In the present application, the content of the compound represented by Formula (1) in the non-aqueous electrolyte may vary within a wide range, for example, it may be 0.001-5 wt% based on the total weight of the non-aqueous electrolyte. Preferably, the content of the compound represented by Formula (1) in the non-aqueous electrolyte is 0.001-3 wt% based on the total weight of the non-aqueous electrolyte.

In the non-aqueous electrolyte of the lithium ion battery of the present application, the battery performance may be significantly improved as long as the compound represented by the Formula (1) is contained in a very small amount. In order to further improve the effect, the content of the compound represented by the Formula (1) may be appropriately increased, but it should not exceed 5% by weight, because when the content of the compound represented by the Formula (1) in the non-aqueous electrolyte is higher than 5% by weight, the performance of the lithium ion battery would not be further improved. On the contrary, it may be adversely affected, because excessive compound represented by Formula (1) would increase the side reaction in battery, consume the content of active lithium, make the SEI film formed at the negative electrode too thick, increase the battery impedance, and deteriorate the battery cycle performance.

In the present application, the content of the vinylene carbonate may be determined according to the total weight of the non-aqueous electrolyte, for example, in the non-aqueous electrolyte, the content of vinylene carbonate is 0.1-5 wt% based on the total weight of the non-aqueous electrolyte. Preferably, the content of vinylene carbonate in the non-aqueous electrolyte is 0.5-3 wt% based on the total weight of the non-aqueous electrolyte; more preferably, the content of vinylene carbonate in the non-aqueous electrolyte is 1-3 wt% based on the total weight of the non-aqueous electrolyte. When the content of vinylene carbonate is lower than this range, the effect is not obvious. When the content of vinylene carbonate is higher than this range, the internal resistance of battery would be increased and the battery cycle performance would be deteriorated.

In the present application, the organic solvent in the non-aqueous electrolyte of the lithium ion battery may be various organic solvents commonly used in the field for preparation of non-aqueous electrolyte, and is not particularly limited. For example, the organic solvent may be selected from one or more of cyclic carbonate, linear carbonate and carboxylic ester.

The cyclic carbonate used for non-aqueous electrolysis of lithium ion batteries may include one or more of vinylene carbonate, propylene carbonate and ethylene carbonate.

The linear carbonate used for non-aqueous electrolysis of lithium ion batteries may include one or more of dimethyl carbonate, diethyl carbonate and ethyl methyl carbonate.

The carboxylic ester used for non-aqueous electrolysis of lithium ion batteries may include one or more of methyl acetate, ethyl acetate, methyl propionate, ethyl propionate, methyl butyrate, methyl isobutyrate, methyl trimethylacetate and ethyl trimethylacetate.

The ether used for non-aqueous electrolysis of lithium ion batteries may include one or more of ethylene glycol dimethyl ether, 1,3-dioxolane and 1,1,2,2-tetrafluoroethyl-2,2,3,3-tetrafluoropropyl ether.

In a preferred embodiment of the present application, the organic solvent is a mixture of ethylene carbonate and ethyl methyl carbonate. When the organic solvent is a mixture of ethylene carbonate and ethyl methyl carbonate, the non-aqueous electrolyte may have higher dielectric constant and lower viscosity, thus improving the overall performances of battery.

According to the present application, the lithium salt in the non-aqueous electrolyte for lithium ion batteries may adopt various lithium salts commonly used in the field for preparation of lithium ion batteries, and there is no particular limitation. For example, it may be selected from one or more of $LiPF_6$, $LiBF_4$, LiBOB, LiDFOB, $LiPO_2F_2$, $LiSbF_6$, $LiAsF_6$, $LiN(SO_2CF_3)_2$, $LiN(SO_2C_2F_5)_2$, $LiC(SO_2CF_3)_3$ and LiFSI; preferably, the lithium salt is selected from one or more of $LiPF_6$, $LiPO_2F_2$ and LiFSI; and more referably, the lithium salt is $LiPF_6$. When the above lithium salts are used, the electrochemical stability, conductivity and graphite negative electrode compatibility of the non-aqueous electrolyte may be improved.

In the present application, the content of the lithium salt may be the usual content in the field of lithium ion batteries, and there is no particular limitation. In the application, the content of the lithium salt in the non-aqueous electrolyte for lithium ion batteries is 0.1-2 mol/L. Preferably, the content of the lithium salt in the non-aqueous electrolyte for lithium ion batteries is 0.8-1.5 mol/L. More preferably, the content of the lithium salt in the non-aqueous electrolyte for lithium ion batteries is 0.8-1.2 mol/L. When the content of lithium salt is within this range, the non-aqueous electrolyte may possess better viscosity and conductivity.

In the present application, the non-aqueous electrolyte of lithium ion batteries contains not only the compound represented by Formula (1) and vinylene carbonate, but also various other additives commonly used in the field to improve the performance of lithium ion battery, such as 1,3-propane sultone, 1,4-butane sultone, methylene methane disulfonate, fluoroethylene carbonate, ethylene sulfate, methylene methyl disulfonate, lithium difluorophosphate, lithium difluoro(oxalato)borate, tris (trimethylsilane) phosphate, propene sultone, fluorobenzene and vinyl ethylenecarbonate; preferably, the other additives are one or more of lithium difluorophosphate, ethylene sulfate and methylene methanedisulfonate. When the other additives mentioned above are added, the high-temperature storage and cycle performances of battery may be further improved.

In the present application, the content of other additives in lithium ion batteries may be the conventional content of various additives in the field. For example, the content of other additives may be 0.1-5 wt% of the total weight of the non-aqueous electrolyte of lithium ion batteries. Preferably, the content of additive may be 0.1-3 wt% of the total weight of the non-aqueous electrolyte of lithium ion batteries. More preferably, the content of other additive may be 0.3-1 wt% of the total weight of the non-aqueous electrolyte of lithium ion batteries.

According to the application, the active material of the positive electrode of lithium ion batteries is $LiFePO_4$. The active material of negative electrode may be selected from various materials commonly used in the field of lithium ion batteries, and is not particularly limited. For example, it may be one or more of metallic lithium, graphite-like carbon materials, hard carbon materials, soft carbon materials, silicon-based, tin-based, antimony-based, aluminum-based, transition metal compounds, silicon-carbon materials and silicon-oxygen-carbon. Preferably, the active material of negative electrode is selected from one or more of artificial graphite, natural graphite, modified natural graphite and silicon oxygen carbon.

In the present application, the preparation of positive and negative electrodes of lithium ion batteries may be conducted according to the method commonly used in the field for positive and negative electrodes of lithium ion batteries, and there is no special limitation. For example, the active materials of positive and negative electrodes may be mixed with conductive agent and adhesive, the mixture may be dispersed in organic solvent to prepare a slurry, and then the slurry obtained is coated on a current collector, then dried and calendared. The adopted conductive agent, adhesive, organic solvent and current collector may be materials and substances commonly used in the field, which are not described in details here.

According to the present application, preferably, the compacted density of the material of the positive electrode is 2-3g/$cm^3$.

According to the present application, a compacted density of a material of the negative electrode is above 1.3g/$cm^3$; and more preferably, the compacted density of the material of the negative electrode is 1.3-1.8g/$cm^3$.

According to the present application, a voltage range of the lithium ion battery is 2-4V; more preferably, the voltage range of the lithium ion battery is 2-3.65 V.

According to the present application, an liquid injection coefficient of the lithium ion battery is 2-7g/Ah; preferably, the liquid injection coefficient of the lithium ion battery is 3-5g/Ah. Therefore, not only can the battery have good high-temperature cycle and storage performances, but also the use amount of electrolyte may be reduced, thus saving the production cost of battery.

According to the present application, the separator placed between the positive electrode and negative electrode may be made of various materials commonly used as diaphragms in the field, and there is no particular limitation. For example, it may be selected from one or more of polyolefin diaphragm, polyamide diaphragm, polysulfone diaphragm, polyphosphazene diaphragm, polyether sulfone diaphragm, polyether ether ketone diaphragm, polyether amide diaphragm and polyacrylonitrile diaphragm. In a particularly preferred embodiment of the present application, the separator is selected from polyethylene diaphragm, and it is a polyethylene microporous diaphragm.

In the present application, the preparation of lithium ion batteries may be conducted with a sandwich method commonly used in the field. For example, a separator is placed between a positive electrode plate and a negative electrode plate coated with active materials, and then the whole lithium ion battery is wound, then the wound body is squashed and put into a packaging bag for vacuum baking and drying to obtain a battery core, and then electrolyte is injected into the battery core, vacuum packaged and allowed to set, then the battery core is formed. This is a well-known method in the field and would not be described here.

The present application will be described in details below with embodiments. In the following embodiments, unless otherwise specified, all materials used are commercially available.

In the following embodiments and comparative examples, compounds 1-6 were purchased from Shanghai Aladdin Biochemical Technology Co., Ltd.

$LiFePO_4$, the positive electrode active material, was purchased from Shenzhen Defang Nanotechnology Co., Ltd., and the model was DY-3.

The test methods of each performance in the following embodiments and comparative examples were conducted according to the following test examples.

Test Example 1: High-temperature cycle performance test

The lithium ion batteries prepared in the following embodiments and comparative examples were placed in an oven with a constant temperature of 45°C, charged to 3.65V at 1C constant current, then charged at constant voltage until the current dropped to 0.1C, and then discharged at 1C constant current to 2.0V, and so on for 2500 times, recording the discharge capacity for the first cycle and the 2500th cycle, and calculating the capacity retention rate of high-temperature cycle according to the following formula:

Capacity retention rate (%) = Discharge capacity of the 2500th cycle / Discharge capacity of the first cycle ×100%.

Test Example 2: High-temperature storage performance test

lithium ion batteries prepared in the following examples and comparative examples were charged to 3.65V at room temperature with constant current and constant voltage of 1C, then charged at constant voltage until the current dropped to 0.1C, and the initial discharge capacity and initial battery size were measured. After being stored at 60°C for 30 days, they were discharged to 2.0V at 1C, and the retention capacity, recovery capacity and battery size after storage were measured, and the battery capacity retention rate, capacity recovery rate and size expansion rate were calculated as follows:

Capacity retention rate (%) = Retention capacity / Initial discharge capacity ×100%;

Capacity recovery rate (%) = Recovery capacity / Initial discharge capacity ×100%;

Size expansion rate (%) = (Battery size after storage - Initial battery size) / Initial battery size ×100%.

Test Example 3: Iron ion precipitation test

Disassemble the battery after 2500 cycles, take out the battery negative electrode and separator of negative electrode side, and dissolve them in a mixed solution of $HNO_3$ (concentration: 14.5mol/L) and $H_2O$ (mixed according to the weight ratio of 1:2). After complete dissolution, 20g of dissolved solution was divided into 50mL reagent bottle, and then the amount of iron ions precipitated on the negative electrode and separator was measured by Inductively Coupled Plasma Optical Emission Spectroscopy (ICP-OES).

Embodiment 1

1) Preparation of electrolyte

Ethylene carbonate (EC) and ethyl methyl carbonate (EMC) were mixed according to the weight ratio of EC: EMC = 3: 7, then lithium hexafluorophosphate ($LiPF_6$) was added to the obtained mixture until the molar concentration was 1mol/L, and then Compound 1, which was 0.001 wt% of the total weight of the electrolyte, was added (note: Compound 1 here was Compound 1 described in the present specification, the same below) and 2 wt% VC of the total weight of the electrolyte.

2) Preparation of positive plate

Uniformly mixing the positive electrode active material $LiFePO_4$, conductive carbon black Super-P and binder polyvinylidene fluoride (PVDF) according to the weight ratio of 93:4:3, and then dispersing them in N-methyl-2-pyrrolidone (NMP) to obtain a positive electrode slurry. Uniformly coating the positive electrode slurry on both sides of aluminum foil, drying, calendering (the compacted density is shown in Table 1) and vacuum drying, and then an aluminum lead-out wire was welded by an ultrasonic welder to obtain a positive electrode plate to obtain a positive electrode plate with a thickness of 100±2 μm.

3) Preparation of negative plate

Uniformly mixing the negative active material of modified natural graphite, conductive carbon black Super-P, and binder styrene-butadiene rubber (SBR) and carboxymethyl cellulose (CMC) according to the weight ratio of 95:1:2.5:1.5, and then dispersing the mixture in deionized water to obtain a negative electrode slurry. Coating the negative electrode slurry on both sides of copper foil, drying, calendering (the compacted density is shown in Table 1) and vacuum drying, and then a nickel lead-out wire was welded by an ultrasonic welder to to obtain a negative electrode plate with a thickness of 120±2 μm.

4) Preparation of battery core

A three-layer separator film (star source material, 12+2+2 um ceramic PP separator) with a thickness of 20 μm was placed between the positive plate and negative plate, and then the sandwich structure consisting of the positive electrode plate, negative electrode plate and separator was wound, and then the wound body was put into an aluminum foil packaging bag and baked in vacuum at 75°C for 48 hours to obtain a battery core to be injected with liquid.

5) Liquid injection and formation of battery core

In a glove box with a dew point below -40°C, the electrolyte prepared in Step 1 was injected into the battery prepared in Step 4, wherein the injection coefficient is 4g/Ah, and then the formation of the first charging was conducted according to the following steps: charged at 0.05C constant current for 180min, charged at 0.1C constant current for 180min, allowed to set for 24hr and sealed, and then charged to 3.65V at 0.2C constant current to 2.0V.

Embodiments 2-18 and Comparative Examples 1-8

Most of the steps are the same as Embodiment 1, except that the compacted density of positive and negative materials of lithium ion battery, the type and amount of compound represented by Formula (1) added in electrolyte, the amount of VC added and the type and amount of other additives are different, and the specific contents are shown in Table 1.

Table 1

| Type | Positive electrode material compacted density | Negative electrode material compacted density | Compound represented by Formula (1) and its content (wt%) | VC content (wt%) | Other additives and contents (wt%) |
|---|---|---|---|---|---|
| Embodiment 1 | 2.5g/cm$^3$ | 1.6 g/cm$^3$ | | VC: 2 | / |
| Embodiment 2 | 2.5g/cm$^3$ | 1.6 g/cm$^3$ | | VC: 2 | / |
| Embodiment 3 | 2.5g/cm$^3$ | 1.6 g/cm$^3$ | | VC: 2 | / |
| Embodiment 4 | 2.5g/cm$^3$ | 1.6 g/cm$^3$ | | VC: 2 | / |
| Embodiment 5 | 2.5g/cm$^3$ | 1.6 g/cm$^3$ | | VC: 2 | / |
| Embodiment 6 | 2.5g/cm$^3$ | 1.6 g/cm$^3$ | | VC: 2 | / |
| Embodiment 7 | 2.5g/cm$^3$ | 1.6 g/cm$^3$ | | VC: 2 | / |
| Embodiment 8 | 2.5g/cm$^3$ | 1.6 g/cm$^3$ | | VC: 2 | / |
| Embodiment 9 | 2.5g/cm$^3$ | 1.6 g/cm$^3$ | | VC: 2 | / |

(continued)

| Type | Positive electrode material compacted density | Negative electrode material compacted density | Compound represented by Formula (1) and its content (wt%) | VC content (wt%) | Other additives and contents (wt%) |
|---|---|---|---|---|---|
| Embodiment 10 | 3.0g/cm$^3$ | 1.6 g/cm$^3$ | | VC: 2 | / |
| Embodiment 11 | 2.0g/cm$^3$ | 1.6 g/cm$^3$ | | VC: 2 | / |
| Embodiment 12 | 2.5g/cm$^3$ | 1.6 g/cm$^3$ | | VC: 1 | / |
| Embodiment 13 | 2.5g/cm$^3$ | 1.6 g/cm$^3$ | | VC: 3 | / |
| Embodiment 14 | 2.5g/cm$^3$ | 1.8 g/cm$^3$ | | VC: 2 | / |
| Embodiment 15 | 2.5g/cm$^3$ | 1.3 g/cm$^3$ | | VC: 2 | / |
| Embodiment 16 | 2.5g/cm$^3$ | 1.6 g/cm$^3$ | | VC: 2 | DTD: 0.5 |
| Embodiment 17 | 2.5g/cm$^3$ | 1.6 g/cm$^3$ | | VC: 2 | MMDS: 0.5 |
| Embodiment 18 | 2.5g/cm$^3$ | 1.6 g/cm$^3$ | | VC: 2 | $LiPO_2F_2$: 0.5 |
| Comparative Example 1 | 2.5g/cm$^3$ | 1.6g/cm$^3$ | / | VC: 2 | / |
| Comparative Example 2 | 2.5g/cm$^3$ | 1.6g/cm$^3$ | / | VC: 2 | DTD: 0.5 |
| Comparative Example 3 | 2.5g/cm$^3$ | 1.6g/cm$^3$ | / | VC: 2 | MMDS: 0.5 |
| Comparative Example 4 | 2.5g/cm$^3$ | 1.6g/cm$^3$ | / | VC: 2 | $LiPO_2F_2$: 0.5 |
| Comparative Example 5 | 3.0g/cm$^3$ | 1.6g/cm$^3$ | / | VC: 2 | / |
| Comparative | 2.0g/cm$^3$ | 1.6g/cm$^3$ | / | VC: 2 | / |
| Example 6 | | | | | |
| Comparative Example 7 | 2.5g/cm$^3$ | 1.6g/cm$^3$ | | / | / |
| Comparative Example 8 | 2.5g/cm$^3$ | 1.8g/cm$^3$ | / | VC: 2 | / |
| Note: / indicates that no corresponding substance is added, $LiPO_2F_2$ is lithium difluorophosphate, DTD is ethylene sulfate, and MMDS is methylene methanedisulfonate. | | | | | |

The related performances of lithium ion batteries prepared in Embodiments 1-18 and Comparative Examples 1-8 are shown in Table 2.

Table 2

| Type | High-temperature cycle performance | High-temperature storage performance (After 30 days of storage at 60°C) | | | Fe ion precipitation test result |
|---|---|---|---|---|---|
| | The 2500th cycle capacity retention rate (%) at 45°C/1C | Capacity retention rate | Capacity recovery rate | Size expansion rate | |
| Embodiment 1 | 78.1% | 78.5% | 80.3% | 20.1% | 105.3ppm |
| Embodiment 2 | 80.1% | 80.5% | 82.2% | 18.9% | 99.9ppm |
| Embodiment 3 | 82.9% | 83.0% | 85.6% | 15.8% | 78.9ppm |
| Embodiment 4 | 82.4% | 82.4% | 84.9% | 16.2% | 81.6ppm |
| Embodiment 5 | 82.1% | 82.0% | 84.33% | 16.0% | 84.5ppm |
| Embodiment 6 | 84.6% | 86.2% | 88.0% | 14.6% | 75.1ppm |
| Embodiment 7 | 88.4% | 89.1% | 91.0% | 10.2% | 50.5ppm |
| Embodiment 8 | 86.4% | 87.6% | 89.5% | 12.2% | 60.4ppm |
| Embodiment 9 | 85.6% | 86.3% | 88.9% | 12.9% | 65.7ppm |
| Embodiment 10 | 83.3% | 84.6% | 86.5% | 16.0% | 83.7ppm |
| Embodiment 11 | 82.5% | 84.1% | 85.9% | 16.9% | 87.9ppm |
| Embodiment 12 | 82.7% | 83.2% | 85.6% | 14.8% | 78.7ppm |
| Embodiment 13 | 83.9% | 84.5% | 86.9% | 14.4% | 70.7ppm |
| Embodiment 14 | 81.5% | 81.6% | 83.9% | 18.6% | 97.7ppm |
| Embodiment 15 | 86.8% | 87.9% | 89.8% | 12.0% | 56.4ppm |
| Embodiment 16 | 89.4% | 90.0% | 92.1% | 9.2% | 44.8ppm |
| Embodiment 17 | 88.9% | 89.5% | 91.4% | 9.5% | 47.4ppm |
| Embodiment 18 | 89.7% | 90.5% | 92.6% | 8.5% | 40.5ppm |
| Comparative Example 1 | 77.2% | 76.4% | 78.6% | 25.3% | 130.4ppm |
| Comparative Example 2 | 77.7% | 77.5% | 78.8% | 22.5% | 115.7ppm |

(continued)

| Type | High-temperature cycle performance | High-temperature storage performance (After 30 days of storage at 60°C) | | | Fe ion precipitation test result |
|---|---|---|---|---|---|
| | The 2500th cycle capacity retention rate (%) at 45°C/1C | Capacity retention rate | Capacity recovery rate | Size expansion rate | |
| Comparative Example 3 | 77.4% | 76.9% | 78.9% | 24.0% | 119.2ppm |
| Comparative | 77.9% | 78.0% | 79.8% | 20.9% | 110.4ppm |
| Example 4 | | | | | |
| Comparative Example 5 | 75.6% | 74.2% | 76.5% | 32.1% | 168.1ppm |
| Comparative Example 6 | 76.8% | 76.1% | 78.3% | 28.1% | 145.2ppm |
| Comparative Example 7 | 50.8% | 53.4% | 55.6% | 100.6% | 255.9ppm |
| Comparative Example 8 | 77.6% | 78.0% | 80.2% | 23.2% | 126.1ppm |

From the results of the above Embodiments 1-9 and Comparative Example 1, it can be seen that with the positive electrode active material $LiFePO_4$ of the present application, when the non-aqueous electrolyte of lithium ion batteries contains both VC and the compound represented by Formula (1) provided by the present application, the storage and cycle performances of the lithium ion battery at high temperature can be improved to varying degrees, and the precipitation of iron ions can be effectively suppressed.

It can be seen from Embodiment 7, Embodiments 10-11, Comparative Example 1 and Comparative Examples 5-6 that, when the compacted density of positive electrode material is within the range defined by the present application, the high-temperature cycle and storage performance of lithium ion batteries can be significantly improved by adding both VC and the compound represented by Formula (1) into the non-aqueous electrolyte of lithium ion batteries.

From the results of Embodiments 1, 2, 7 and 9 and Comparative Example 1, it can be seen that the high-temperature storage and cycle performances of battery can be effectively improved when the positive electrode active material defined by the present application is adopted and the compacted density of the positive electrode material is within the range defined by the present application, or the addition amount of the compound represented by Formula (1) in the electrolyte is between 0.001-5 wt%. When the content is 0.5 wt%, it can improve the performance of the battery best. When the content is less than 0.5 wt%, the residual amount in the battery is not enough, so the improvement of battery performance is not significant. When the content is more than 0.5 wt%, the residual amount of the compound in the electrode plate would also increase, and excessive compound represented by Formula (1) would increase side reaction in the battery, which is not conducive to the improvement of battery cycle and other performances.

From the results of Embodiment 7, Embodiments 14-15 and Comparative Example 8, it can be seen that when the compacted density of the positive electrode material is within the range defined by the present application, the compound represented by Formula (1) and VC are both added to the non-aqueous electrolyte, and the compacted density of negative electrode material is controlled within the range of 1.3-1.8g/$cm^3$, the high-temperature cycle and storage performances of lithium ion battery would be excellent.

From the results of Embodiment 7, Embodiments 16-18, Comparative Example 1 and Comparative Examples 2-4, it can be seen that, when the compacted density of the positive electrode material is within the range defined by the present application, the high-temperature cycle and storage performances of lithium ion batteries can be further improved by adding both the compound represented by Formula (1) and additives such as $LiPO_2F_2$, DTD and MMDS to the non-aqueous electrolyte of lithium ion batteries. However, when the compound represented by Formula (1) is not added to the non-aqueous electrolyte, even if other additives are added, the high-temperature cycle and storage performances of lithium ion batteries would not be significantly improved.

From the results of Comparative Example 7 and Embodiment 7, it can be seen that when the positive electrode active material defined by the present application is adopted and the compacted density of the positive electrode material is within the range defined by the present application, only adding the compound represented by Formula (1) without VC would not significantly improve the high-temperature cycle and storage performances of lithium ion batteries.

Fig. 1 is a SEM image of negative electrode of lithium ion battery after 2500 cycles at 45°C, according to Embodiment 7 of the present application. Fig. 2 is a SEM image of negative electrode of lithium ion battery after 2500 cycles at 45°C, according to Comparative example 1 of the present application. As can be seen from Fig. 1 and Fig. 2, compared with Comparative Example 1, i. e., when only 2 wt% of VC was added to the electrolyte, there were obvious lithium dendrites (circled in Fig. 2) on the negative electrode plate after 2500 cycles, while in Embodiment 7, i. e., when 0.5 wt% of the compound represented by Formula (1) was added with 2 wt% of VC, there were almost no lithium dendrites on the negative electrode after 2500 cycles. Therefore, with the positive electrode active material and the range of compacted density of the positive electrode material defined by the present application, on the basis of adding VC, the compound represented by Formula (1) can obviously inhibit the formation of lithium dendrites.

The preferred embodiments of the present application have been described in detail above, but the present application is not limited thereto. Within the technical concept of the present application, various simple modifications may be made to the technical solutions of the present application, including the combination of various technical features in any other suitable way. These simple modifications and combinations shall also be regarded as the contents disclosed by the present application and belong to the protection scope of the present application.

## Claims

1. A lithium ion battery, comprising a positive electrode, a negative electrode, a separator arranged between the positive electrode and the negative electrode, and a non-aqueous electrolyte;

   an active material of the positive electrode comprises $LiFePO_4$,
   the non-aqueous electrolyte comprises an organic solvent, a lithium salt, vinylene carbonate and a compound represented by Formula (1):

   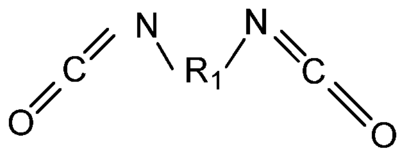

   Formula (1)

   in formula (1), $R_1$ is one or more of chain, cyclic and aromatic group with 2-20 carbon atoms; and
   a compacted density of the active material of the positive electrode is more than 2g/cm$^3$.

2. The lithium ion battery according to claim 1, wherein in Formula (1), $R_1$ is one or more of chain, cyclic and aromatic group with 3-18 carbon atoms;
   preferably, $R_1$ is selected from one or more of following structures, * represents binding position:

   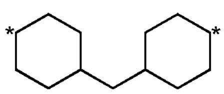 , 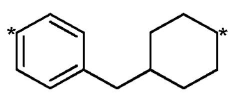 , 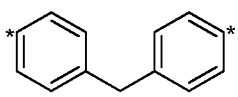 ,

   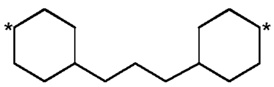 , 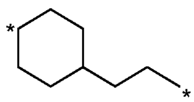 , 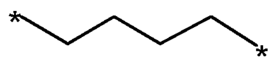 ,

   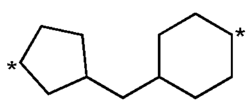 , 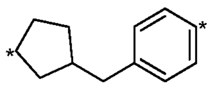 , 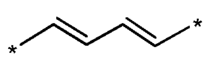 ,

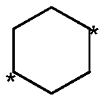, 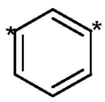 and 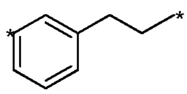.

3. The lithium ion battery of claims 1 or 2, wherein the compound represented by Formula (1) is selected from one or more of following compounds:

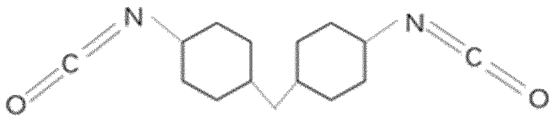
Compound 1,

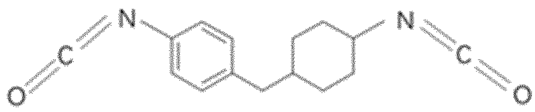
Compound 2,

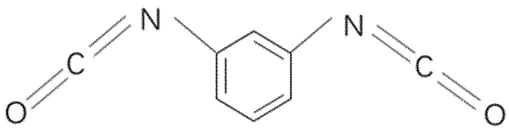
Compound 3,

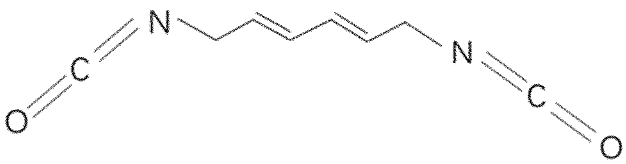
Compound 4,

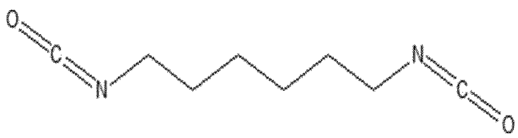
Compound 5,

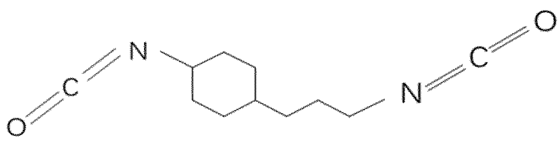
Compound 6,

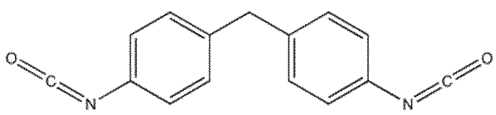
Compound 7

and

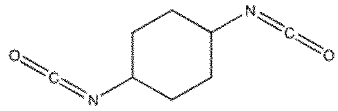

Compound 8.

4. The lithium ion battery of any claims of 1-3, wherein a content of the compound represented by Formula (1) in the non-aqueous electrolyte is 0.001-5 wt% based on a total weight of the non-aqueous electrolyte;
preferably, the content of the compound represented by Formula (1) in the non-aqueous electrolyte is 0.001-3 wt% based on the total weight of the non-aqueous electrolyte.

5. The lithium ion battery of any claims of 1-3, wherein the content of vinylene carbonate in the non-aqueous electrolyte is 0.1-5 wt% based on the total weight of the non-aqueous electrolyte;
preferably, the content of the vinylene carbonate in the non-aqueous electrolyte is 0.5-3 wt% based on the total weight of the non-aqueous electrolyte.

6. The lithium ion battery of any claims of 1-3, wherein the organic solvent is one or more of cyclic carbonate, linear carbonate, carboxylic ester and ether;

preferably, the cyclic carbonate comprises one or more of vinylene carbonate, propylene carbonate and ethylene carbonate;
preferably, the linear carbonate comprises one or more of dimethyl carbonate, diethyl carbonate and ethyl methyl carbonate;
preferably, the carboxylic ester comprises one or more of methyl acetate, ethyl acetate, methyl propionate, ethyl propionate, methyl butyrate, methyl isobutyrate, methyl trimethylacetate and ethyl trimethylacetate;
preferably, the ether comprises one or more of ethylene glycol dimethyl ether, 1,3-dioxolane and 1,1,2,2-tetrafluoroethyl-2,2,3,3-tetrafluoropropyl ether; and
more preferably, the organic solvent is a mixture of ethylene carbonate and ethyl methyl carbonate.

7. The lithium ion battery of any claims of 1-3, wherein the lithium salt is one or more of $LiPF_6$, $LiBF_4$, LiBOB, LiDFOB, $LiPO_2F_2$, $LiSbF_6$, $LiAsF_6$, $LiN(SO_2CF_3)_2$, $LiN(SO_2C_2F_5)_2$, $LiC(SO_2CF_3)_3$ and LiFSI;

preferably, the lithium salt is one or more of $LiPF_6$, $LiPO_2F_2$ and LiFSI;
preferably, the content of the lithium salt in the non-aqueous electrolyte is 0.1-2 mol/L; and
more preferably, the content of the lithium salt in the non-aqueous electrolyte is 0.8-1.5 mol/L.

8. The lithium ion battery of any claims of 1-3, wherein the non-aqueous electrolyte further comprises other additives, and the other additives are 1,3-propane sultone, 1,4-butane sultone, methylene methane disulfonate, fluoroethylene carbonate, ethylene sulfate, methylene methyl disulfonate, lithium difluorophosphate, lithium difluoro(oxalato)borate, tris (trimethylsilane) phosphate, propene sultone, fluorobenzene and vinyl ethylenecarbonate;

preferably, the other additives are one or more of lithium difluorophosphate, ethylene sulfate and methylene methanedisulfonate;
preferably, the content of the other additives in the non-aqueous electrolyte is 0.1-5 wt% based on the total weight of the non-aqueous electrolyte; and
more preferably, the content of the other additives in the non-aqueous electrolyte is 0.1-3 wt% based on the total weight of the non-aqueous electrolyte.

9. The lithium ion battery of any claims of 1-3, wherein the compacted density of the active material of the positive electrode is 2-3g/$cm^3$;

preferably, a compacted density of a material of the negative electrode is above 1.3g/$cm^3$; and
more preferably, the compacted density of the material of the negative electrode is 1.3-1.8g/$cm^3$.

10. The lithium ion battery of any claims of 1-3, wherein an liquid injection coefficient of the lithium ion battery is 2-7g/Ah;

preferably, the liquid injection coefficient of the lithium ion battery is 3-5g/Ah;
preferably, a voltage range of the lithium ion battery is 2-4V; and
more preferably, the voltage range of the lithium ion battery is 2-3.65V.

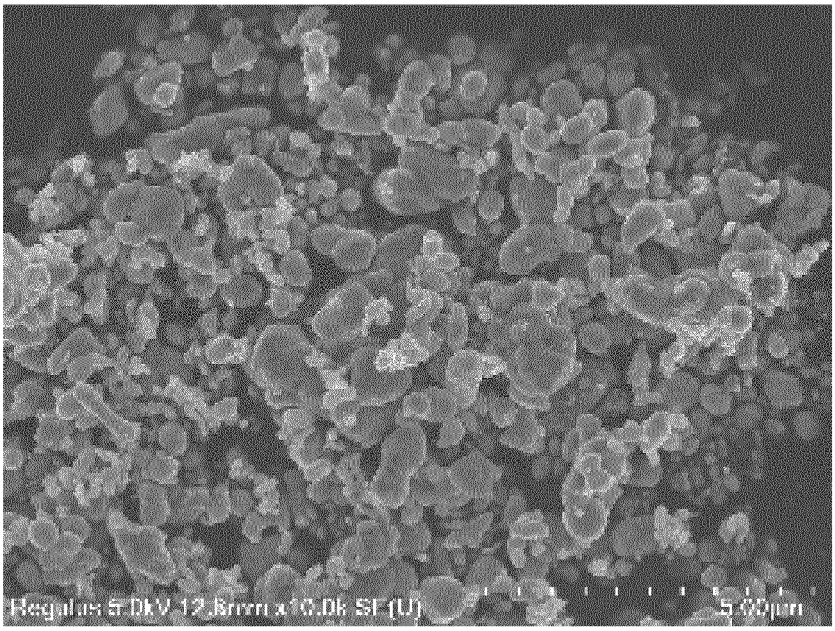


Fig. 1

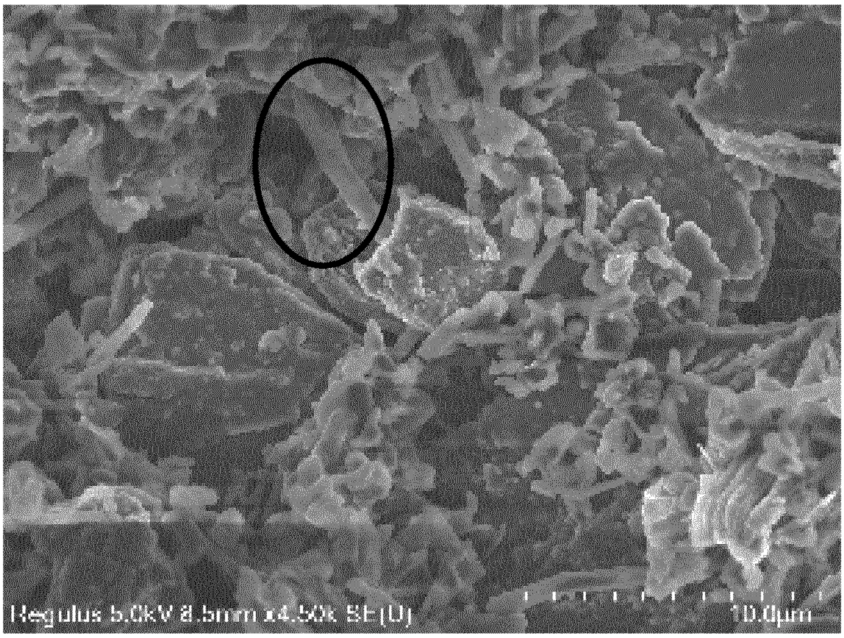


Fig. 2

**INTERNATIONAL SEARCH REPORT**

International application No.
**PCT/CN2021/131060**

**A. CLASSIFICATION OF SUBJECT MATTER**

H01M 10/0567(2010.01)i; H01M 4/58(2010.01)i; H01M 10/0525(2010.01)i

According to International Patent Classification (IPC) or to both national classification and IPC

**B. FIELDS SEARCHED**

Minimum documentation searched (classification system followed by classification symbols)

H01M

Documentation searched other than minimum documentation to the extent that such documents are included in the fields searched

Electronic data base consulted during the international search (name of data base and, where practicable, search terms used)

CNABS; CNTXT; WPABSC; VEN; ENTXT; ENTXTC; CNKI; STN: 电池, 正极活性材料, 磷酸铁锂, LiFePO4, 异氰酸酯, 压实密度, battery, cell, positive electrode active material, lithium iron phosphate, isocyanate, compaction density

**C. DOCUMENTS CONSIDERED TO BE RELEVANT**

| Category* | Citation of document, with indication, where appropriate, of the relevant passages | Relevant to claim No. |
|---|---|---|
| X | CN 102754268 A (MITSUBISHI CHEMICAL CORPORATION) 24 October 2012 (2012-10-24)<br>description paragraphs 93-372, 481-794 | 1-10 |
| X | CN 111628217 A (TOYOTA MOTOR CO., LTD. et al.) 04 September 2020 (2020-09-04)<br>description paragraphs 40-227, 263-309, 430-499 | 1-10 |
| X | CN 111937215 A (DAIKIN INDUSTRIES, LTD.) 13 November 2020 (2020-11-13)<br>description paragraphs 211-398, 472-518, 640-706 | 1-10 |
| A | JP 2013038072 A (MITSUBISHI CHEMICAL CORPORATION) 21 February 2013 (2013-02-21)<br>entire document | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 January 2022** | **29 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/131060**

| Patent document cited in search report | Publication date (day/month/year) | Patent family member(s) | Publication date (day/month/year) |
|---|---|---|---|
| CN 102754268 A | 24 October 2012 | KR 20120133378 A | 10 December 2012 |
| | | KR 101412899 B1 | 26 June 2014 |
| | | KR 20160043149 A | 20 April 2016 |
| | | KR 101726144 B1 | 11 April 2017 |
| | | CN 102754268 B | 19 November 2014 |
| | | US 2012308881 A1 | 06 December 2012 |
| | | US 8673489 B2 | 18 March 2014 |
| | | KR 20140007000 A | 16 January 2014 |
| | | KR 101612351 B1 | 15 April 2016 |
| | | CN 104167564 A | 26 November 2014 |
| | | JP 2016029668 A | 03 March 2016 |
| | | JP 6187566 B2 | 30 August 2017 |
| | | US 2013280622 A1 | 24 October 2013 |
| | | US 9515348 B2 | 06 December 2016 |
| | | JP 2013152956 A | 08 August 2013 |
| | | JP 5831493 B2 | 09 December 2015 |
| | | JP 2011187440 A | 22 September 2011 |
| | | JP 5353923 B2 | 27 November 2013 |
| | | WO 2011099585 A1 | 18 August 2011 |
| | | EP 2958181 A1 | 23 December 2015 |
| | | EP 2958181 B1 | 14 June 2017 |
| | | EP 2535976 A1 | 19 December 2012 |
| | | EP 2535976 B1 | 02 September 2015 |
| | | CN 104167564 B | 12 April 2017 |
| CN 111628217 A | 04 September 2020 | US 2020274200 A1 | 27 August 2020 |
| | | JP 2020140826 A | 03 September 2020 |
| | | JP 6831409 B2 | 17 February 2021 |
| | | EP 3703171 A1 | 02 September 2020 |
| CN 111937215 A | 13 November 2020 | US 2021043974 A1 | 11 February 2021 |
| | | KR 20200121358 A | 23 October 2020 |
| | | EP 3764451 A1 | 13 January 2021 |
| | | JP WO2019188210 A1 | 14 January 2021 |
| | | WO 2019188210 A1 | 03 October 2019 |
| JP 2013038072 A | 21 February 2013 | JP 6031856 B2 | 24 November 2016 |

Form PCT/ISA/210 (patent family annex) (January 2015)